# EUROPEAN PATENT SPECIFICATION

(11) **EP 1 764 097 B1**
(45) Date of publication and mention of the grant of the patent: **16.03.2011**
(21) Application number: 06116917.3
(22) Date of filing: 10.07.2006
(51) Int. Cl.: A61K 31/395, A61K 31/64, A61K 31/435, A61P 11/02, A61P 1/02

(54) **Methods and compositions for soothing oral and nasal tissues**
Methoden und Zusammensetzungen zur Beruhigung von Mund- und Nasenschleimhäuten
Méthodes et compositions pour soulager les muqueuses buccales et nasales

(30) Priority: 12.08.2005 EP 05017601
(43) Date of publication of application: 21.03.2007
(73) Proprietor: The Procter & Gamble Company, Cincinnati, OH 45202 (US)
(72) Inventor: Keenan, Siobhan, Ascot, SL5 8PZ (GB); Bermin, Jenny, Staines, Middlesex TW18 3DP (GB); Tonstrom, Kristofer, S-112 31, Stockholm (SE)
(74) Representative: Clemo, Nicholas Graham

(56) References cited:
- EP-A- 1 520 850
- WO-A-2004/043906

## Description

### FIELD OF THE INVENTION

The present invention relates to the use of a combination of cooling agents and salivating agents for the preparation of a medicament for soothing irritated nasal tissues.

### BACKGROUND OF THE INVENTION

Many examples exist of medicaments designed to relieve or sooth irritated oral and nasal tissues. These medicaments typically rely upon a pharmaceutically active ingredient such as benzocaine which is a local anaesthetic. These ingredients, whilst on the whole successful, are sometimes not able to effectively sooth the nasal tissues and the nasopharyngeal region. Furthermore, the effects of pharmaceutically active ingredients such as these are sometimes considered by consumers as being more than is necessary to achieve the required results. A need exists for medicaments that provide a soothing effect in a moderate manner.

Soothing of irritated oral and nasal tissues may occur via increased moisturisation of the tissues. However, many ingredients suitable for increasing moisturisation of the oral and nasal tissues have noted drawbacks. For example, citric acid is able to increase oral salivation around the area of the tongue; yet is not able to improve moisturisation in the entirety of the oral cavity and does not modulate nasal moisturisation. This tends to prevent citric acid from being able to provide notable soothing effect in the nasophayngeal area. Furthermore, levels of citric acid that are capable of producing noticeable benefits around the tongue may taste acidic or astringent, and may also negatively impact product stability due to the high hygroscopicity of citric acid.

A need exists to provide medicaments that successfully sooth irritated oral and/or nasal tissues without associated aesthetic negatives and/or product stability issues.

WO 2004/043906 discloses that trans-pellitorin is a salivating agent that can be used in foodstuffs and oral hygiene products and that it can minimize the dry mouth feel from eating certain snacks. EP-A-1520850 discloses other alkadienamides and their use in a range of compositions, including nasal compositions.

### SUMMARY OF THE INVENTION

The present invention provides for the use of a salivating agent according to formula (I): 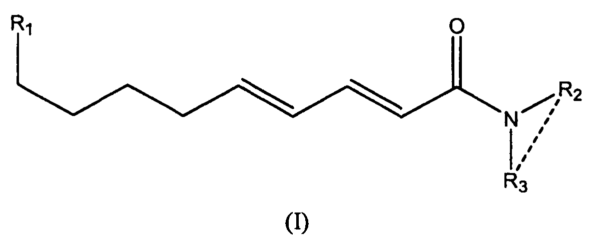 wherein R₁ represents C1-C2 n-alkyl; R₂ is 2-methyl-1-propyl and R₃ is hydrogen, or R₂ and R₃ taken together is a moiety having the formula ―(CH₂)ₙ-wherein n is 4 or 5, or mixtures thereof in the preparation of a medicament for soothing irritated nasal tissues by modulating nasal secretions. The medicament further comprises
a cooling agent selected from menthol, peppermint oil, N-substituted-p-menthane-3-carboxamides, acyclic tertiary and secondary carboxamides, 3-1-menthoxy propan-1,2-diol, monomenthyl glutarate and mixtures thereof.

### DETAILED DESCRIPTION OF THE INVENTION

Unless otherwise stated herein, all percentages are weight percentages. Unless otherwise stated herein, all measurements are taken at 25°C.

As used herein, "oral" includes the buccal cavity, tongue and the throat, and "nasal" includes the nose, nasal cavity and the nasopharynx. As used herein, "soothing" includes relaxing, moisturizing, relieving, reducing pain and the like. Oral and nasal secretion includes salivation, moisturisation and mucosal secretion, preferably in the nasal passage, nasal conchae, pharynx, nasopharynx, oral cavity, soft palate and the tongue.

The salivating agent of the present invention comprises a material according to formula (I): 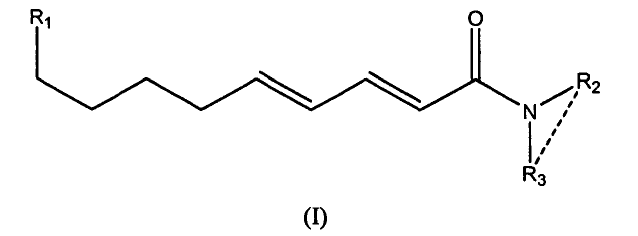 wherein R₁ represents C1-C2 n-alkyl; R₂ is 2-methyl-1-propyl and R₃ is hydrogen, or R₂ and R₃ taken together is a moiety having the formula -(CH₂)ₙ- wherein n is 4 or 5, or mixtures thereof. Preferably, the salivating agent comprises a material wherein R₂ is 2-methyl-1-propyl and R₃ is hydrogen, more preferably wherein R₁ is C1 n-alkyl, R₂ is 2-methyl-1-propyl and R₃ is hydrogen. More preferably, the salivating agent comprises trans-pellitorin, a chemical having a structure according to formula (II):

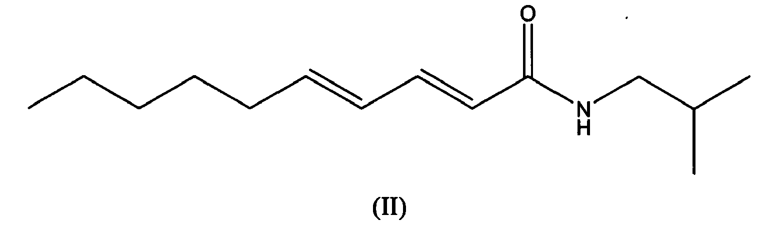

Methods of making the salivating agents suitable for use herein are described in US2004/0241312 A1 and EP1520850A2.

Without wishing to be bound by theory it is believed that the salivating agents herein have surprisingly been found to sooth and relieve irritated oral and/or nasal tissues when applied thereupon. It is believed that this effect is associated with the action of the salivating agents modulating oral and/or nasal secretions, whilst not impacting the aesthetics or stability of the medicament.

Furthermore, the salivating agents as described herein provide improved long-lasting moisturisation in the oral and/or nasal tissues, in comparison with organic acids such as citric acid. Without wishing to be bound by theory, this is believed to be due to two factors. In the first instance, it is believed that the lipophilic nature of the salivating agents herein enable the compounds to adhere to the mucosal tissues present in the oral and nasal tissues. This adherence enable continued stimulation of oral and/or nasal secretions, especially where the secretions themselves may wash the salivating agent away, for example such as is the case with organic hydrophilic acids such as citric acid. Furthermore, it is believed that the salivating agents herein actually stimulate oral and/or nasal secretions via nerve stimulation, rather than via osmotic pressure, as is the case with traditional salivating agents. Without wishing to be bound by theory, it is believed that the salivating agents herein stimulation secretion in the oral and/or nasal tissues via secondary processes transduced by interaction of trigeminal and parasympathetic nerves. Thus the salivating agents herein are only perceived in the oral cavity where they are ingested, but the combination of their lipophilic nature which enables increased residency time, and the stimulatory pathway, enables prolonged stimulation of secretion from both the oral and nasal tissues, driving improved moisturisation and soothing of the oral and nasal tissues. This is in comparison with traditional salivating agents that only stimulate oral secretion, and not driving relief and soothing in the nasal tissues.

Preferably, the medicament comprises from 0.01% to 5% salivating agent by weight of the medicament, preferably from 0.01% to 2%, more preferably from 0.01 % to 1.5%, even more preferably from 0.02% to 1.0%.

The medicament of the present invention may be in any suitable form, including confectionery, chewing gum, throat and cough lozenge, throat disc, cough syrup and the like. Preferably the medicament is in the form of a confectionery. Suitable confectionery forms include hard boiled sweets, soft boiled sweets, chewing gums, gummy-based sweets, centre-fill confectionery, or lollies. The confectionery compositions of the present invention preferably take the form of a hard boiled sweet.

The compositions of the present invention are preferably in the form of a hard boiled candy or gum based confectionary. Hard boiled candies are sugar- or sugar substitute-based compositions wherein the base is formed into a candy mass with cooking and subsequently formed into a drop and allowed to cool. The candy mass once cooled forms a glassy matrix that contains the salivating agent therein. Once formed, the hard boiled candies preferably have a water level of about 0.1% to about 4% by weight of the composition. Gum based confectionary are soft to semi-solid compositions which are sugar or sugar-substitute based, wherein a suitable gelling agent is cooked with the sugar or sugar-substitute and water to achieve the right consistency, and either deposited into moulds or extruded into a continuous rope & cut.

A suitable sugar base for a hard candy comprises from about 30% to about 85% glucose syrup and from about 15% to about 70% sucrose. Alternatively, a sugar-free base can be used for the shell. Suitable sugar-free bases include bulk sweeteners such as isomalt, maltitol, sorbitol and xylitol. Isomalt and maltitol are preferred as bulk sugar-free bases. Xylitol is preferred as an ancillary base, preferably being present in sugar-free candies at a level of from about 0.1 % to about 5%.

Other preferred forms of the medicament of the present invention are cough lozenges and cough syrups. Cough lozenges are sugar-based solid or semi-solid compositions, preferably in the form of hard boiled candies and/or gummies. A suitable cough lozenge comprises from about 30% to about 50% of glucose syrup and from about 15% to about 75% of sucrose. Cough lozenges may further comprise honey, honey derivatives and/or honey flavours or lenitive herbs. in concentration from about 0.05% to 10%, preferably from 0.1% to 5%.

Cough syrups are sugar-based liquid composition further comprising additional active ingredients, such guaifenesin. Cough syrups comprise from about 25% to about 65% of sucrose. Additionally, from about 30% to about 45% of glucose syrup may be further comprised in the formulation of cough syrups.

The medicament of the present invention further comprises a cooling composition and, optionally, a warming composition. The cooling and warming composition may be present in the medicament separately, or at the same time. When used at the same time, the cooling and warming compositions are preferably located in distinct and discrete regions within the medicament and are preferably adapted to provide sequential release profiles. As used herein, adapted to provide sequential release profiles' means that the compositions are chemically and/or physically modified relative to a homogeneous mix of the compositions. It will be understood that many such medicaments will release the warming or cooling agent over the period of ingestion of the product and that there may be some simultaneous perception of warming agent and cooling agent.

An essential component of the cooling composition is a physiological cooling agent. Suitable levels of the cooling agent are from 0.001 to 10%, preferably from 0.01 to 5%, more preferably from 0.01 to 2%, more preferably still from 0.01 to 0.5% by weight of the medicament. A test for physiological cooling agents is described in GB-A-1,452,291, published Oct. 13, 1976.

Suitable physiological cooling agents are described in WO97/06695. Those for use herein are physiological cooling agents selected from menthol, peppermint oil, N-substituted-p-menthane-3-carboxamides, acyclic tertiary and secondary carboxamides, 3-1-menthoxy propan-1,2-diol, monomenthyl glutarate and mixtures thereof. The carboxamides found most useful are those described in U.S. Pat. No. 4,136,163, Jan. 23, 1979 to Watson et al., and U.S. Pat. No. 4,230, 688, Oct. 28, 1980 to Rowsell et al. The carboxamides in U.S. Pat. No. 4,136,163 are N-substituted-p-menthane-3-carboxamides, such as N-ethyl-p-menthane-3-carboxamide, commercially available as WS-3 from Wilkinson Sword. The carboxamides of U.S. Pat. No. 4,230,688 are certain acyclic tertiary and secondary carboxamides, such as trimethyl isopropyl butanamide, commercially available as WS-23 from Wilkinson Sword. More preferred for use herein are monomenthyl glutarate, N-ethyl-p-menthane-3-carboxamide, trimethyl isopropyl butanamide and mixtures thereof, more preferably still monomenthyl glutarate, commercially available as Ultracool 2 from IFF (Netherlands). The balance of the cooling composition may be made up of a suitable appropriate carrier, such as water, propylene glycol or a bulk sweetener, described in more detail below. The cooling composition can further comprise a warming agent as described herein provided that the predominant effect is one of cooling.

The medicaments of the present invention may further comprise a warming composition. An essential component of the warming composition is a physiological warming agent. Suitable levels of the warming agent are from about 0.001 to about 10%, preferably from about 0.005 to about 5%, more preferably from about 0.01 to about 1%, more preferably still from about 0.01% to about 0.5% by weight of the throat drop.

Physiological warming agents can be tested for using a modification of the test for cooling agents described above, the test being modified to use benzyl alcohol rather than menthol as the reference sample. Preferred physiological warming agents are those selected from the group consisting of vanillyl alcohol n-alcohol isobutyl ether, vanillyl alcohol n-amino ether, vanillyl alcohol isoamyl ether, vanillyl alcohol n-hexyl ether, vanillyl alcohol methyl ether, vanillyl alcohol ethyl ether, gingerol, shogaol, paradol, zingerone, capsaicin, dihydrocapsaicin, nordihydrocapsaicin, homocapsaicin, homodihydrocapsaicin, ethanol, iso-propyl alcohol, iso-amylalcohol, benzyl alcohol, chloroform, eugenol, cinnamon oil, cinnamic aldehyde, phosphate derivatives thereof, and mixtures thereof. The phosphate derivatives mentioned are those described in WO 97/02273. A commercial example of a suitable warming agent for use herein is Optaheat (Symrise, Germany). The balance of the warming composition may be made up of a suitable appropriate carrier, such as water, propylene glycol or a bulk sweetener, described in more detail below. The warming composition can further comprise a cooling agent as described herein provided that the predominant effect is one of warming.

The medicaments herein can also include a flavouring agent. As used herein, the term 'flavouring agent' means those flavour essences and equivalent synthetic ingredients which are added to the flavour composition for the principal purpose of providing flavour to the confectionery product. It excludes warming and cooling agents as described above. Flavouring agents well known in the confectionery art can be added to the flavour compositions of the invention. These flavouring agents can be chosen from synthetic flavouring liquid and/or oils derived from plants leaves, flowers, fruits and so forth, and combinations thereof. Representative flavouring liquids include: artificial, natural or synthetic fruit flavours such as eucalyptus, lemon, orange, banana, grape, lime, apricot and grapefruit oils and fruit essences including apple, strawberry, cherry, orange, pineapple and so forth; bean and nut derived flavours such as coffee, cocoa, cola, peanut, almond and so forth; and root derive flavours such as licorice or ginger. The amount of flavouring agent employed is normally a matter of preference subject to such factors as flavour type, base type and strength desired. In general, amounts up to about 4% by weight are usable with amounts of from about 0.1 % to about 1% being preferred.

The medicaments of the present invention may further comprise other active ingredients such as local anaesthetics, antitussives, decongestants and the like. The medicaments may also comprise pH adjusting agents and buffers in order to control the pH and hence the stability of the medicament. Other optional ingredients include organic acids including citric, ascorbic, malic, tartaric acids, or mixtures thereof. Where the medicament is the form of a water-based syrup, the medicament may further comprise thickening agents that impart an increased viscosity to the liquid formulation.

Without wishing to be bound by theory it is believed that the medicaments of the present invention provide improved soothing of oral and/or nasal tissues by a synergistic action of modulating oral and/or nasal secretions and improved cooling and soothing sensation associated with the cooling agent. The two ingredients act together to provide an improved composition that soothes and relieves irritated oral and/or nasal tissues.

The following examples are given to illustrate the compositions and uses according to the invention. However, the invention is not limited thereto.

### Sugar-Free Hard Boiled Candy

### Example 1

### 1. Preparation of premixes

### a) MEDICATION PREMIX

| DESCRIPTION | MAN% w/w |
|---|---|
| FLAVOUR | 38.33 |
| LEVOMENTHOL EP | 12.77 |
| MONOMENTHOL GLUTARATE | 24.33 |
| OPTAFLOW (TRANS-PELLITORIN) | 19.46 |
| ALCOHOL 96% | 5.11 |
| Total | 100.00 |

Combine the above ingredients, heating if required up to a maximum of 20°C and stir until fully homogeneous.

### b) ACESULFAME K/COLOUR SOLUTION

| | |
|---|---|
| WATER | 81.39 |
| ACESULFAME K | 10.47 |
| BETA-CAROTINE 1% CWS E160A | 8.14 |
| Total | 100.00 |

Add the Acesulfame K and the colour ingredient to the water and mix until homogeneous.

### c) XYLITOL SOLUTION

| DESCRIPTION | MAN% w/w |
|---|---|
| WATER | 60.00 |
| XYLITOL | 40.00 |
| Total | 100.00 |

Xylitol is dissolved in water and mixed until homogeneous.

### 2. Making process

Isomalt Liquid (93.7%) and xylitol solution (3.8%) are weighed and added to the cooker. The mixture is cooked to 148°C applying a vacuum of -0.92 bar minimum after cooking. To the hot candy base, the following ingredients are added by weight of the composition: Acesulfame K/colour solution (0.4%), citric acid anhydrous (1.2%), ascorbic acid (0.6%), medication premix (0.4%), aspartame (0.05%) and flavours (2%).

The medicated/coloured candy mass is then transferred onto the cooling belt to cool down the mass. A thin film of trennwax is used to ensure separation of the mass from the cooling belt. The mass is transferred through batch former, rope former and punching equipment to form drops. Talc is used to prevent the mass/drops from sticking to the forming equipment. The drops are cooled down on the cooling belt passing the cooling tunnel to obtain their final consistency for packing.

### Sugar-based Hard Boiled Candy

### Example 2

### 1. Preparation of premixes

### a) MEDICATION PREMIX

| DESCRIPTION | MAN% w/w |
|---|---|
| FLAVOUR | 23.13 |
| LEVOMENTHOL EP | 16.51 |
| MONOMENTHYL GLUTARATE | 30.72 |
| OPTAFLOW (TRANS-PELLITORIN) | 24.57 |
| ALCOHOL 96% | 5.07 |
| Total | 100.00 |

Combine the ingredients of the medication pre-mix, heating if required up to a maximum of 20°C and stir until fully homogeneous.

### b) COLOUR SOLUTION

| | Man %w/w |
|---|---|
| WATER | 68.182 |
| BETACAROTINE 1% CWS (160A) | 31.818 |
| Total | 100.00 |

To the water add the colour ingredient(s) under stirring at ambient temperature and stir until completely dissolved.

### 2. Making process

Water (16.1%), sucrose (48.4%), glucose liquid (36.3%) and an antifoam agent are weighed and added to the cooker. The mixture is cooked to 148°C applying a vacuum of -0.92 bar minimum after cooking. To the hot candy base, the following ingredients are added (per 100 kg of candy mass): β carotene solution (0.1%), citric acid anhydrous (1.2%), ascorbic acid (0.6%), and medication premix (0.3%) and flavour (2%).

The medicated/coloured candy mass is then transferred onto the cooling belt to cool down the mass. A thin film of trennwax is used to ensure separation of the mass from the cooling belt. The mass is transferred through batch former, rope former and punching equipment to form drops. Talc is used to prevent the mass/drops from sticking to the forming equipment. The drops are cooled down on the cooling belt passing the cooling tunnel to obtain their final consistency for packing

### Gelatine based gummy

### Example 3

### a) Cooked gelatine base:

| DESCRIPTION | MAN% w/w |
|---|---|
| WATER | 16.54% |
| GELATINE 250 BLOOM | 5.26% |
| SUGAR | 45.11% |
| GLUCOSE SYRUP | 33.08% |
| C₁₆-C₁₈ VEGETABLE FAT | 0.01% |
| Total | 100.00 |

Premix the water, gelatine and vegetable fat into a premix vessel. Add sugar and glucose syrup until homogeneous. Cook the mix using a jet cooker at 120-130°C and a vacuum of 3.2 - 3.8 bar. Dispense the cooked gelatine base into a finishing vessel , keep at 70-80°C.

### b) Gummy finishing:

| DESCRIPTION | MAN% w/w |
|---|---|
| COOKED GELATINE BASE | 95.80% |
| OPTAFLOW (TRANS-PELLITORIN) | 0.08% |
| MONOMETHYL GLUTARATE | 0.30% |
| FLAVOUR | 0.12% |
| FRUIT JUICE CONCENTRATE | 2.00% |
| CITRIC ACID MONOHYDRATE | 1.50% |
| COLOURANT | 0.20% |
| Total | 100.00 |

Add to cooked gelatine base at 70-80°C the flavouring, fruit juice concentrate, citric acid and colourant. Mix until fully homogeneous. Transfer to filling line and deposit into pre-moulded starch trays.

Leave the gummies to cure in the starch moulds for 24 hours at 25°C until gelled, for a harder consistency leave gummies to cure for longer.

Once cured de-mould the gummies and separate from the starch. Polish the gummies in a coating drum with a vegetable wax for a smooth finish.

### Example 4

### 1. Preparation of premixes

### a) MEDICATION PREMIX

| DESCRIPTION | Man% w/w |
|---|---|
| FLAVOUR | 66.04 |
| MONOMENTHYL GLUTARATE | 18.87 |
| OPTAFLOW (TRANS-PELLITORIN) | 15.09 |
| Total | 100.00 |

Combine the ingredients of the medication pre-mix, heating if required up to a maximum of 20°C and stir until fully homogeneous.

### b) COLOUR SOLUTION

| | Man %w/w |
|---|---|
| WATER | 95.00 |
| FOOD COLOUR LIGHT GREEN | 5.00 |
| Total | 100.00 |

To the water add the colour ingredient(s) under stirring at ambient temperature and stir until completely dissolved.

### 2. Making process

Water (16.1%), sucrose (48.4%), glucose liquid (36.3%) and an antifoam agent are weighed and added to the cooker. The mixture is cooked to 148°C applying a vacuum of -0.92 bar minimum after cooking. To the hot candy base, the following ingredients are added (per 100 kg of candy mass): light green colour solution (0.1%), citric acid (0.8%), malic acid (0.4%), medication premix (0.5%) and flavour (2.0%).

The medicated/coloured candy mass is then transferred onto the cooling belt to cool down the mass. A thin film of trennwax is used to ensure separation of the mass from the cooling belt. The mass is transferred through batch former, rope former and punching equipment to form drops. Talc is used to prevent the mass/drops from sticking to the forming equipment. The drops are cooled down on the cooling belt passing the cooling tunnel to obtain their final consistency for packing

### Example 5

### Cough lozenge

| DESCRIPTION | Man% w/w |
|---|---|
| SUCROSE | 62.3 |
| GLUCOSE LIQUID 80% | 43.3 |
| OPTAFLOW (TRANS-PELLITORIN) | 0.08 |
| FLAVOUR | 0.1 |
| LEVOMENTHOL | 0.2 |
| SIMETHICONE EMULSION 30% | 0.0075 |
| ETHANOL | 0.1 |
| VEGETABLE OIL & WAX | 0.025 |
| DEXTROMETHORPHAN BASE | 0.2 |
| HONEY OR/AND HONEY FLAVOUR | 2.6 |
| PURIFIED WATER | UP TO 100 |

## Claims

1. The use of a salivating agent according to formula (I): 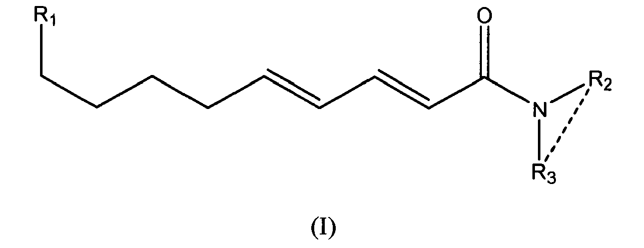 wherein R₁ represents C₁-C₂ n-alkyl; R₂ is 2-methyl-1-propyl and R₃ is hydrogen, or R₂ and R₃ taken together is a moiety having the formula -(CH₂)ₙ- wherein n is 4 or 5, or mixtures thereof in the preparation of a medicament for soothing the nasal tissues by modulating nasal secretions,
wherein the medicament further comprises a cooling agent selected from menthol, peppermint oil, N-substituted-p-menthane-3-carboxamides, acyclic tertiary and secondary carboxamides, 3-1-menthoxy propan-1,2-diol, monomenthyl glutarate and mixtures thereof.

2. The use according to claim 1, wherein the salivating agent comprises trans-pellitorin.

3. The use according to any one of the preceding claims wherein the medicament comprises from 0.01% to 5% salivating agent by weight of the medicament, preferably from 0.01% to 2%, more preferably from 0.01% to 1.5%, even more preferably from 0.02% to 1.0%.

4. The use according to any one of the preceding claims wherein the medicament is in the form of a confectionery, preferably a hard boiled sweet.

5. The use according to any one of the preceding claims wherein the cooling agent comprises monomenthyl glutarate.

## Patentansprüche

1. Verwendung eines Speichelabsonderungsmittels nach Formel (I): 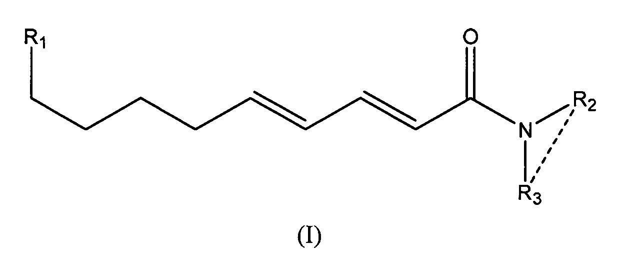 worin R₁ für C₁-C₂-n-Alkyl steht, R₂ 2-Methyl-1-propyl ist und R₃ Wasserstoff ist oder R₂ und R₃ zusammen genommen eine Einheit mit der Formel -(CH₂)ₙ-sind, worin n gleich 4 oder 5 ist, oder Mischungen davon bei der Herstellung eines Medikaments zur Beruhigung des Nasengewebes durch Modulieren von Nasensekreten,
wobei das Medikament ferner ein Kühlmittel umfasst, das ausgewählt ist aus Menthol, Pfefferminzöl, N-substituierten p-Menthan-3-carboxamiden, azyklischen tertiären und sekundären Carboxamiden, 3-1-Menthoxypropan-1,2-diol, Monomenthylglutarat und Mischungen davon.

2. Verwendung nach Anspruch 1, wobei das Speichelabsonderungsmittel trans-Pellitorin umfasst.

3. Verwendung nach einem der vorstehenden Ansprüche, wobei das Medikament zu 0,01 Gew.-% bis 5 Gew.-%, vorzugsweise zu 0,01 Gew.-% bis 2 Gew.-%, bevorzugter zu 0,01 Gew.-% bis 1,5 Gew.-%, noch bevorzugter zu 0,02 Gew.-% bis 1,0 Gew.-% des Medikaments Speichelabsonderungsmittel umfasst.

4. Verwendung nach einem der vorstehenden Ansprüche, wobei das Medikament in Form einer Süßware, vorzugsweise eines Hartbonbons vorliegt.

5. Verwendung nach einem der vorstehenden Ansprüche, wobei das Kühlmittel Monomenthylglutarat umfasst.

## Revendications

1. Utilisation d'un agent de salivation selon la formule (I) : 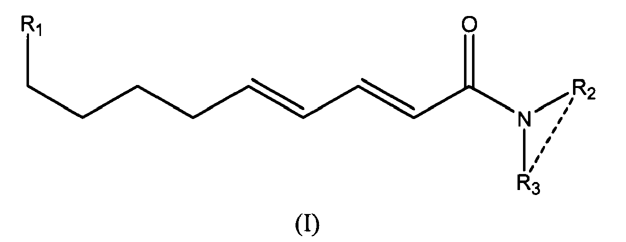 dans laquelle R₁ représente un n-alkyle en C₁ à C₂ ; R₂ est le 2-méthyl-1-propyle et R₃ est un hydrogène, ou R₂ et R₃ pris conjointement sont un fragment de formule -(CH₂)ₙ- dans laquelle n est 4 ou 5, ou leurs mélanges dans la préparation d'un médicament pour apaiser les tissus nasaux en modulant les sécrétions nasales,
dans laquelle le médicament comprend en outre un agent de refroidissement choisi parmi le menthol, l'essence de menthe poivrée, les p-menthane-3-carboxamides N-substitués, les carboxamides acycliques tertiaires et secondaires, le 3-1-menthoxy propan-1,2-diol, le glutarate de monomenthyle et leurs mélanges.

2. Utilisation selon la revendication 1, dans laquelle l'agent de salivation comprend de la trans-pellitorine.

3. Utilisation selon l'une quelconque des revendications précédentes, dans laquelle le médicament comprend de 0,01 % à 5 % d'agent de salivation en poids du médicament, de préférence de 0,01 % à 2 %, plus préférablement de 0,01 % à 1,5 %, encore plus préférablement de 0,02 % à 1,0 %.

4. Utilisation selon l'une quelconque des revendications précédentes, dans laquelle le médicament est sous la forme d'une confiserie, de préférence un bonbon dur bouilli.

5. Utilisation selon l'une quelconque des revendications précédentes, dans laquelle l'agent de refroidissement comprend du glutarate de monomenthyle.
